# EUROPEAN PATENT SPECIFICATION

(11) **EP 1 065 217 B1**
(45) Date of publication and mention of the grant of the patent: **24.09.2003**
(21) Application number: 99112439.7
(22) Date of filing: 30.06.1999
(51) Int. Cl.: C08B 37/00, C08B 31/04, C08B 3/22, C08B 3/12, C08B 3/14, C08B 37/16, A01C 1/00, C09K 17/00

(54) **Polysaccharide aspartate**
Polysaccharidaspartat
Aspartate de polysaccharide

(43) Date of publication of application: 03.01.2001
(73) Proprietor: Südzucker Aktiengesellschaft, 68165 Mannheim (DE)
(72) Inventor: Kretzschmar, Gerhard, Dr., 65760 Eschborn (DE)

(56) References cited:
- US-A- 3 671 184
- CHEMICAL ABSTRACTS, vol. 129, no. 23, 7 December 1998 (1998-12-07) Columbus, Ohio, US; abstract no. 310134, "Synthesis and characterization of beta-cyclodextrin derivatives containing amino groups and carboxyl groups." XP002124415 & DAI RONGJI ET AL.: BEIJING LIGONG DAXUE XUEBAO BIANJIBU, vol. 18, no. 2, 1998, pages 159-164, china

## Description

### Background:

The present invention relates to new biopolymers derived from polysaccharides as renewable resources (RR). According to a definition given in the *Dictionary of Renewable Resources* H. Zoebelein, Editor, VCH Weinheim, 1997, page XIII), RR are products originated from plants and animals used for industrial purposes (energy, functional applications and chemical modification) and include also food products that are not used for nutrition, as well as wastes and co-products for food processing. For chemical applications, petrochemical-based products are dominant but there are other areas where RR-based materials have their domain, and there is a large overlap in between being largely dependent on prices and performance of the respective materials in distinct applications.

It is generally accepted that new products, cheaper and more ecologically oriented processes as well as new areas of application would promote the use of RR, thus increasing the importance and ecological advantages of RR-derived products. By using a moderate degree of derivatisation of the RRs, materials close to the natural products can be produced that often fit better in biological degradation cycles than an entirely synthetic product. Polysaccharides are the most abundant RR being polymers of simple monomeric units, hexoses or pentoses, linked by glycosidic bonds. The monomeric groups may be arranged in long, linear chains (e.g. amylose, cellulose, starch) or may be branched (amylopectin, pectins). According to their most important biological functions, the polysaccharides may be classified into the
- skeletal polysaccharides which serve as mechanically rigid building structures (e.g. cellulose, hemicellulose, pectins, agar, chitin),
- nutrient polysaccharides which act as metabolic reserves (e.g. starches, glycogen, guar gum),
- protecting polysaccharides as exudates of higher plants (e.g. gum arabic, tragacanth) or as exopolysaccharides of bacterial growth (dextran, xanthan).
- small cyclic polysaccharides like α-, β-, or γ-cyclodextrins

Most native polysaccharides are hydrophilic in character due to some O-functionality present. E.g. the versatility of the polysaccharides is highly increased by introduction of neutral, acidic, alkaline substituents, by cross-linking, by oxidation, by copolymerisation or by physical modification (Reviews: W.Burchard, *Polysaccharide, Eigenschaften und Nutzung,* Springer Verlag, Berlin, Heidelberg, NY, Tokyo, 1985; Ullmann (5.) A25, 21-23).

The known chemistry of polysaccharides can be summarised using starch as a reference which exhibits chemical reactivity mainly based on the primary and secondary OH groups and/or the glycosidic α-1,4- and α-1,6- linkages, the primary OH groups being the most reactive towards electrophilic reagents. The literature and patents describing state of the art in this area, especially the organic and inorganic esters which can be obtained from starches by alkylation and esterification were compiled by E.S.Lower (*Riv. Ital. Sostanze Grasse,* 1996, 73(4), 159-163).

As already mentioned, a moderate degree of derivatisation of the polysaccharide would produce materials close to the natural products providing advantages with respect to biodegradability, biocompatibility and ecology. This advantage could even be enhanced by using a chemical modifier itself being a natural product, e.g. a natural amino acid. However, prior art does not provide means to modify a polysaccharide by covalent bonding to amino acids while leaving both the polysaccharide and the amino acid moieties structurally intact. The known literature only refers to polysaccharides and amino acids either in the context of naturally occuring glycoproteins, certain biochemical systems where both materials are present as chemically unbonded components or as nutritional ingredients. For instance, starch and aspartic acid or an aspartate derivative (aspartame sweetener) are contained in certain foodstuffs (DE 3542905).

### Description of the invention

Surprisingly it has been found that aspartic acid can be covalently bonded to polysaccharides by a simple two-step process comprising (1) the known reaction of the polysaccharide with maleic anhydride and (2) by reaction of the intermediate formed with ammonia thus forming the hitherto unknown polysaccharide aspartates of the general formula (I). 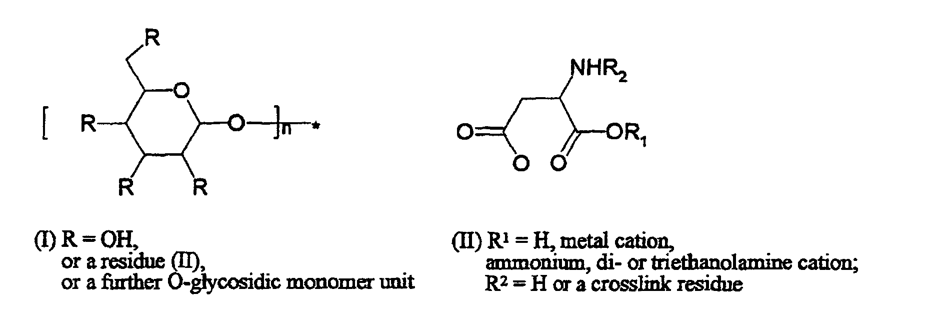 wherein R is hydroxyl, an aspartate residue of formula (II) and/or a glycosidic bond to another monomer sugar unit, and wherein n is greater than one according to the average number of the sugar units given by the parent polysaccharide starting material,
and wherein R¹ is hydrogen, a metal cation, ammonium, di- or triethanolamine cation or a cross-link position to another polysaccharide chain,
and wherein the degree of substitution (DS) with respect to the ratio of the sugar monomeric units being modified with the residues (II) varies from DS 0.001 to DS 3.0.

The DS value is employed as the statistical average to characterise the average DS of the entire quantity which may have a varying rate of DS that may be as little as 0.001, up to the maximum of three. Irrespective of the number of saccharide units, or the actual sequence of substitution. The general formula (I) is intended to represent products where the substitution may occur to various degrees of substitution on all or less than all saccharide units in all or less than all saccharide units. This may be expressed in various ways, for example by referring to an average DS, a varying DS per average saccharide unit, or an average DS per saccharide unit. All such terms include the foregoing concepts.

Furthermore, it has been found that the materials (I) surprisingly are useful as ingredients in soil and seed treatment compositions to promote seedling vigour and plant growth. Since the materials (I) are composed of natural materials only, namely a natural polysaccharide and aspartate, and since the two components are linked *via* enzymatically cleavable ester bonds and glycosidic bonds, they are naturally biodegradable and biocompatible. These features suggest that the materials (I) may be used for a multitude of further technical applications ranging from chromatrography supports, ion exchangers, superabsorbents, complexing agents for heavy metal and calcium ions (scale inhibitor) to drug delivery systems (DDS), to name only a few.

In the following the invention is described in detail:

The term "polysaccharide" as used in the specification and claims is meant to include compounds made up of many, from two to 100 000 or even more monosaccharide units per molecule. Molecules in which only a few monosaccharides (2-100) are linked together are frequently designated as oligosaccharides. However, since this term is not strictly defined, the broader term polysaccharide is uniformly used here, including the small oligosaccharides. In general, these molecules are bonded together by glycoside linkages. Their molecular weights are normally higher than about 340 and range up to millions of Dalton. They are normally naturally occurring molecules like maltose, lactose, sucrose, starch, amylose, glycogen, cellulose, chitin, agar or modified molecules like the dextrins which are prepared by heat treatment or with acids from starches. The polysaccharides have one or more reactive hydroxyl groups per monosaccharide unit and they may be straight, branched chain or cyclic.

The most preferred polysaccharides for the purposes of this invention are starches and starch components like amylose and amylopectin, starch derivatives like dextrin, and cellulose, chitin and chitosan. A broad variety of these polysaccharides is commercially available. Some commercially available starches useful for the purposes of this invention include the following:
- corn starches soluble in cold water: Supramyl 130, Supramyl 131 (Amylum), Cerestar AJ 12014 (Cerestar)
- corn starch water-swellable: Merigel 100 (Amylum)
- corn starch soluble in hot water: Mylbond 100, Mylbond 111, Collofilm 124 (Amylum)
- potato starch soluble in old water: Aeromyl 115 (Sudstarke), Paelli SA2 (Avebe)
- potato starch swellable in cold water: Paselli, WA4 (Avebe)
- wheat starch soluble in hot water: Mylbond 210 (Amylum)
- wheat starch insoluble in water: Meritena 200 (Amylum)
- soluble starches Extramyl M, E, D (Südstärke).

Some commercially available dextrins useful for the purposes of this invention are either white dextrins, yellow dextrins or british gums in dependent on the production process from starches, e.g. the potato dextrins Superior-, Yellow-, Thin-, Medium- or Thick-cooking (Südstärke, Schrobenhausen, Germany). Cyclic dextrins include for example the α-, β- and γ-cyclodextrins available from Aldrich, Fluka or from Wacker (Burghausen, Germany).

Cellulose means any of the convenient and commercially available forms of cellulose such as wood pulp, cotton, hemp, ramie, or regenerated forms such as rayon. In the native state, the glucose chains are extensively hydrogen-bonded, forming microcrystalline regions which are interspersed by amorphous regions. The chemical reactions are known to take place more readily in the amorphous regions. The disruptive effect of the modifications ultimately lead, if carried out to completion, to water-soluble polymers, for instance the known hydroxyethyl cellulose commonly used as adhesives.

The reaction of polysaccharides like starch and cellulose with maleic anhydride has been extensively described. Either aqueous bases or organic solvents were used to manufacture the corresponding polysaccharide maleates. For instance in EP 714914 (Degussa), starch maleates with up to DS ca. 1 were prepared in aqueous base to prepare superabsorbents. In PL 165075 starch and cellulose maleates were manufactured by reacting the polysaccharides with 1-3 mol maleic anhydride (based on one glucose unit) in inert solvents, e.g. in pyridine. Other typical methods include reactions of starch with maleic anhydride in formamide (DE 1941887, Mitsubishi Paper Mills) or acetone/water (JP 045005271, Res. Inst. Prod. Develop.), and reactions of cellulose with maleic anhydride in KOAc/LiCl/dimethylformamide (DMF) (EP 319938, Akzo) or DMF/DMSO/AcNMe₂/N-methylpyrrolidone (JP 8148747, Okura Ind.). Attempts to prepare a β-cyclodextrin maleate from β-cyclodextrin and maleic anhydride toluene or in dimethylformamide as solvents were described in US 3,453,260, whereby only low DS (< 0.2) values of scarcely defined products were achieved, obviously because no base catalysts were employed.
Any of these prior art processes to manufacture a polysaccharide maleate may be used in the present invention. The preferred process for the purpose of this invention uses non-aqueous solvents, the calculated molar amount of maleic anhydride (per monosaccharide unit) to achieve the desired DS value and an equimolar amount (relative to maleic anhydride) of a basic metal salt (MX) like sodium hydrogencarbonate, sodium carbonate, sodium acetate, sodium formiate, lithium acetate or potassium acetate, preferably sodium hydrogencarbonate, sodium carbonate or sodium acetate. The preferred solvents for the purpose of this invention are N,N-dimethylformamide (DMF), N,N-dimethylacetamide, dioxane, N-methylpyrrolidone (NMP), dimethylsulfoxide (DMSO), pyridine, tetrahydrofuran (THF) or mixtures of these solvents, preferably DMF, DMSO and NMP or mixtures of these solvents. The reaction temperature varies from 20°C up to the boiling point of the respective solvent mixture, preferably from 20°C up to 140°C.

The intermediate polysaccharide maleate may be isolated, but the preferred process is to perform the subsequent reaction with ammonia in the same flask and solvents. The reaction of polysaccharide maleates with ammonia has not been described yet.

First attempts to perform this reaction in a solvent-free process with ammonia only gave the half amide of maleic acid under recovery of the polysaccharide. Surprisingly, this cleavage reaction could be suppressed to a large extent by the reaction of ammonia in the same solvent being used to prepare the intermediate polysaccharide maleate. The reaction of the polysaccharide maleate with ammonia is carried out by adding directly 1-5 equivalents (calculated relative to the maleate units) of ammonia to the reaction mixture at 15°C-65°C, preferably at room temperature. At room temperature (ca. 20°C) the reaction time varies from 1-48 h, preferably the reaction is terminated after about 2-18 h and the product precipitated and washed with water and organic solvents, preferably with methanol or ethanol. The counter-ion of the final product (I) may be exchanged by stirring the product with an aqueous solution containing an excess of the respective metal salt, subsequent washing with water and then with methanol or ethanol. The product can be dried in vacuum in an oven. It can be characterised by IR spectroscopy, elemental analysis and saponification of the maleate ester bonds with an excess of a strong base (NaOH, KOH), and then back-titration of the residual base with acid (HCI, H₂SO₄). Only the products (I) having some water solubility can be characterised by NMR. However, the reaction course can be investigated more in detail by using a relatively small saccharide precursor, e.g. sucrose. In this case the NMR and mass spectra can be recorded and assigned.
Typical structures obtained using a starch, amylose or dextrin as precursor polysaccharide are shown in the figure below. (DS = 0.5 on top and a typical cross-link moiety shown below).

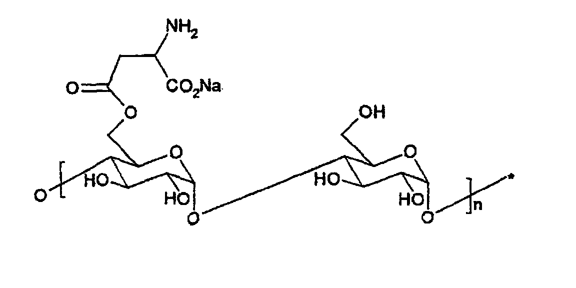

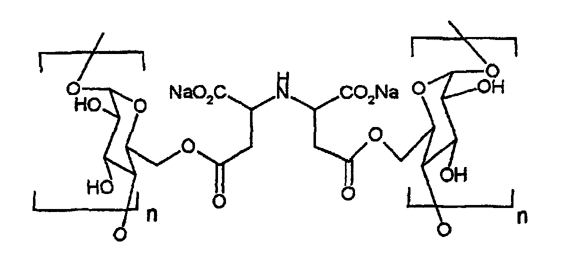

The properties of (I) strongly depend on the DS value: For instance, a low DS value provides relatively more water soluble materials whereas higher DS values give polymers with poor water solubility. Another structural feature of the materials (1) is the presence of some cross-link moieties leading to mostly water-insoluble materials, as illustrated in the figure above. These cross-link moieties are resulting from intermolecular attack of the aspartate nitrogens onto maleate CC-double bonds.

The structure of cross-link moieties was deduced from the structural analysis of the products obtained from sucrose which was subjected to the same reaction sequence of the present invention, as well as from elemental analyses and saponification equivalents required to cleave the aspartate ester bonds. Consistent with these results, a higher degree of cross-linking renders the materials more insoluble in water. Another special feature of the compounds (I) of a relatively low water solubility is their tendency to form solvent gels. The degree of cross-linking can be determined analytically by comparison of the DS value of the intermediate maleate polysaccharide (integration of the of maleate protons in the ¹H NMR spectrum) and the nitrogen content of the final products (I), as determined by elemental analysis.

The structural features in (I), providing either positively (NH₃⁺) or negatively (CO₂⁻) charged groups being aligned along the polymer chain, suggest a multitude of technical applications:

Examples for applications of the compounds (I) include, but are not limited to, superabsorbents, complexing agents (scale inhibitors, ion exchangers, ion sorbents), chromatography supports and drug delivery systems.

Polymer pharmaceutical compositions, in particular drug delivery systems, are based on biocompatible polymers (polyamino acids or polysaccharides) comprising a polymer matrix which releases physically or chemically bonded active ingredients (example: WO 9904824) The new materials (I) provide either ionic (carboxylate or amino) or potential covalent binding sites for the ionic or covalent attachment of pharmaceutically active ingredients. For instance, a linker group or active ingredient carrying a carboxyl group may be attached *via* the amino groups in (I) by virtue of amide bonds, whereas a linker group or active ingredient carrying amino groups may be attached *via* the carboxyl groups in (I) by virtue of amide bonds. Another option is to prepare an aspartate derivative (I) from a cyclodextrin starting material in order to provide a delivery system which retains an active ingredient by inclusion into the cyclodextrin cavity. Cyclodextrins (CyDs) are a homologous family of cyclic polysaccharides that are constructed of α-(1-4)-linked glycopyranose units. The three major CyDs consist of six (α-CyD), seven (β-CyD) and eight (γ-CyD) monosaccharide subunits.

Unmodified and modified CyDs have found widespread medicinal application as carriers for pharmaceuticals, flavours and biochemicals by forming host-guest inclusion complexes (Review: Medicinal Applications of Cyclodextrins, J.Szejtli, Medicinal Research Reviews, *14*, 353, 1994). Applications in general include the complexation of drugs by cyclodextrins, noncomplexing properties in formulations (vehicles), direct therapeutic uses (intraperitoneal dialysis, in shock treatment) and nontherapeutic use, e.g. in diagnostics and chromatography.
In particular, the complexation of problematic drugs and active ingredients which are either poorly soluble, unstable, irritating or difficult to formulate. However, for medicinal purposes only a few CyD derivatives, especially the methylated and β-CyDs, are applicable because of toxicity data and lack of availability in large amounts (J.Szejtli, page 356). It is expected therefore, that CyD aspartates according to the present invention provide a viable alternative to known CyD-derivatives for the following reasons: On the one hand, it was found that the aspartate moieties linked to the CyDs *via* hydrolytically labile ester bonds contribute strongly to improved solubility of the CyD (and its complexes). Surprisingly, the solubility of the β-CyD aspartate prepared according to the present invention (at least 1 g / ml water) surpassed the solubility of the most soluble derivatives hitherto reported (J.Szejtli, page 354). Solubility is of importance at producing liquid drug formulations, or to prevent the formation of insoluble cholesterol complex crystals (nephrotoxicity and hemolysis). Furthermore, the β-CyD aspartate prepared according to the present invention by a very straightforward synthesis (one-flask reaction) is much cheaper than other soluble CyD derivatives being obtained simultaneously with large amounts of toxic wastes as byproducts (J.Szejtli, page 357).

On the other hand, extensive cross-linking *via* the aspartate amino group or *via* remaining maleate moieties may form insoluble, immobilised CyD-containing structures e.g. suitable for chromatographic purposes, to accelerate wound healing in similar manner as cross-linked dextran polymers (Debrisorb®) or to be loaded with drugs or antiseptics. Since the compounds (I), which includes said CyD aspartates, are only composed of natural compounds (polysaccharide and aspartic acid), they are considered to be of little toxicological concern and to be easily biodegradable.

In particular, the materials (I) surprisingly were found to be useful as ingredients in soil and seed treatment compositions to promote seedling vigour and plant growth.

Since (I) is only composed of natural materials (a polysaccharide and an aspartic acid), compounds (I) in general are expected to be easily biodegradable.

The invention will be further described by reference to the following detailed examples. These examples are not meant to limit the scope of the invention that has been set forth in the foregoing description. It should be understood that many variations and modifications may be made while remaining within the scope of the invention.

### Example 1:

100 g (0.617 mol Glc) Supramyl 130 (Amylum Deutschland GmbH), dried at 60°C/1 mbar), was dissolved in N,N-dimethylformamide (1000 ml) at 70°C. After cooling to room temperature, sodium acetate (50.6 g, 0.617 mol) was added, followed by of maleic anhydride (60.5 g, 0.617 mol) in one portion under stirring. Stirring was continued for 15 min at room temperature and 2 h at 80°C. After cooling to room temperature, the mixture was reacted with gaseous ammonia (21.0 g, 1.23 mol). Stirring was continued for 2 h and the mixture was then allowed to stand over night. Ethyl acetate (1000 ml) was added, the mixture stirred for 15 min and the precipitate was collected by filtration, washed with ethyl acetate (2 x 200 ml), and dried at 80°C/20 mbar for 12 h. Yield: 239.2 g crude starch aspartate containing ammonium acetate. The salt was removed by suspending the precipitate in methanol/water (ca. 10 ml/g), stirring for 30 min, and collecting the product by filtration to afford the purified starch aspartate (I) as a product of low water solubility (184 g after drying *i*. *vac.;* 99%).
¹H NMR (Bruker WT 400, D₂O / NaOD; 400 MHz): 2.2-2.7 ppm (m, 2 H, CH₂CO), 3.3-4.2 ppm (m, 7 H, H-2, H-3, H-4, H-5, H-6, H-6', CHNH₂), 5.3-5.5 ppm (bs, 0.9 H, H-1), 5.7-5.8 ppm ( bs, 0.1 H, H-1). IR spectra (Perkin Elmer FT-IR spectrometer Paragon 1000, using an ATR unit): 3250, 3250 (br, CO₂⁻, OH), 2930, 1725 (ester CO), 1627 (CO₂⁻), 1575, 1400, 1348, 1152, 1078, 1002 cm⁻¹. CHN-analysis: C(found) 42.1 %; H(found): 5.2 %; N(found): 2.5 %, Na (found) 2.8%. For a discussion of similar results, see the examples 3-4.

### Example 2:

Potato dextrin (40.0 g, 246 mmol Glc, yellow, medium cooking, from Südstärke) were dissolved in N,N-dimethylformamide (150 ml, Fluka No. 40248) at 50° C. Then maleic anhydride (12.1 g, 123 mmol; Merck No. 800408) and sodium acetate (10.1 g, 123 mmol; Merck No. 106268) were added in portions. The clear solution was stirred for further 4 h at 80° C. After cooling to room temperature, the reaction mixture was poured under stirring into 1-butanol (500 ml, Riedel-de-Häen No. 24124). The precipitate was finally washed with acetone (3x 100 ml, Riedel-de-Häen No. 24201) and then dried for 24 h at 60°C i. vac. to yield 43.0 g of a solid. As expected, the water-soluble maleate dextrin intermediate had a DS value of ca. 0.5, as was determined by ¹H NMR spectroscopy (400 MHz, D₂O, determined by comparison of the integrated olefinic and glucose protons).

### Example 3:

The reaction of potato dextrin (50.0 g, 309 mmol Glc, yellow, medium cooking, from Südstärke) in DMF (400 ml), maleic anhydride (30.3 g, 309 mmol) and sodium acetate (25.3 g, 309 mmol) was performed as described in the previous example 2. The subsequent reaction with ammonia was then performed analogously as described in example 1. The product was precipitated (500 ml ethyl acetate) and dried *i. vac.* to afford the dextrin aspartate (yield 90.9 g, 98%) The crude product was purified by washing in methanol (500 ml, yield 62.5 g). Washing the crude product (10 g) with water (200 ml) and then with methanol and drying *i.vac.* yielded 7.0 g of a purified dextrin aspartate. IR spectrum (Perkin Elmer FT-IR spectrometer Paragon 1000, using an ATR unit): The following characteristic bands not present in the staring material were observed: 1729 (ester CO), 1632 (CO₂⁻) cm⁻¹. CHN-analysis: C(found) 42.2 %; H(found): 6.0 %; N(found): 3.9%; Na (found): 2.8%. This composition fits to a polymer with DS = 1.0 in which about 40% of CO₂H actually-is CO₂Na, which requires C 42.0%, H 5.1.%, N 4.9%, Na 3.2% (for C₁₀H_{14.6}NO₈Na_{0.4}, 284.7 per monomer unit). Some cross-link moieties (= C₁₀H_{14.1}N_{0.5}O₈Na_{0.4}, 285.4) account for the lower nitrogen content observed. After washing the polymer with ammonium chloride, the sodium content was reduced to 0.007% per weight, demonstrating the ion exchanger properties of the product.

### Example 4:

The reaction of potato dextrin (50.0 g, 309 mmol Glc, yellow, medium cooking, from Südstärke) in DMF (400 ml), maleic anhydride (6.10 g, 62 mmol) and sodium acetate (5.10 g, 62 mmol) was performed as described in the previous example 3. The subsequent reaction with ammonia was then performed analogously as described in example 1. The product was precipitated (500 ml ethyl acetate) and dried *i. vac.* to afford the dextrin aspartate (64 g). The crude product was purified by washing in methanol (500 ml, yield 51.4 g). Washing the crude product (10 g) with water (200 ml) and then with methanol and drying *i.vac.* yielded 6.0 g of a purified dextrin aspartate. CHN-analysis: C(found) 42.8 %; H(found): 6.2 %; N(found): 1.8%; Na (found): 1.3%. This composition fits to a polymer with DS = 0.2 in which about 40% of CO₂H actually is CO₂Na, which requires C 43.7%, H 5.8.%, N 1.5%, Na 1.0% (for C₃₄H_{54.6}NO₂₈Na_{0.4}, 934.1 per 5 monomer units). Some cross-link moieties (= C₁₀H_{14.1}N_{0.5}O₈Na_{0.4}, 285.4) account for the lower nitrogen content observed. After washing the polymer with ammonium chloride, the sodium content was reduced to 0.007% per weight, demonstrating the ion exchanger properties of the product.

### Example 5:

Sucrose (60.0 g, 0.175 mol) was dissolved in DMSO (150 ml) at 80°C. After cooling to room temperature, sodium acetate (31.6 g, 0.386 mol) was added, followed by maleic anhydride (37.8 g, 0.386 mol) in one portion under stirring. Stirring was continued for 10 min at room temperature and 2 h at 80°C. After cooling to room temperature, the mixture was reacted with an excess of gaseous ammonia (ca. 20.0 g, 1.11 mol) whereby the temperature initially rose to 60°C. Stirring was continued for 2 h and the mixture was then allowed to stand over night. The viscous honey-like product was pulverised with ethyl acetate dried. A solvent-free product was obtained by freeze-drying from water. The IR spectrum recorded on a Perkin Elmer FT-IR spectrometer (Paragon 1000; ATR unit) showed the following new bands not present in the sucrose IR spectrum: 1726 (ester CO), 1629, 1565 (CO₂⁻) cm⁻¹. The ¹H NMR spectrum of the crude product was recorded on a Bruker WT 400 (400 MHz, D₂O). It showed the presence of an isomeric mixture and the disappearance of the maleate protons (see subsequent example for the assignment of these protons). In addition, the presence of a small amount (ca. 5%) of the half-amide of maleate was detected at δ = 5.90, 6.40 ppm (2d, J = 10 Hz). Mass spectra and MS/MS coupling were performed with a Finnigan LCQ ion trap spectrometer using electrospray ionisation (ESI): Negative mode: [M₁-H]⁻ = 456 (monoaspartate M₁: C₁₆H₂₇NO₁₄; 457); [M₂-H]⁻ = 571 (di-aspartate M₂: C₂₀H₃₂N₂O₁₇; 572); [M₃-H]⁻ = 896 (cross-linked bis-monoaspartate M₃: C₃₂H₅₁NO₂₈; 897). In the positive mode, the respective m/z are at 458, 773 and 898. MS/MS of m/z 897 leads to m/z 554, 456, 341; m/z 571 leads to 456, 438, 341; m/z 554 leads to 456. Elemental analysis of the crude reaction product was the following: C(found) 36.0%, H(found) 6.0%, N(found) 3.1°%, Na (found) 4.8%. The product was further purified by chromatography on Biogel P2 (water) to afford a pleasant, sweet tasting, fluffy white solid. IR: 3230 (OH), 1726 (ester CO), 1620, (CO₂⁻), 1381 cm⁻¹. MS spectroscopy under the conditions as described above (negative mode) showed predominantly m/z = 572 (C₂₀H₃₂N₂O₁₇; 572). Elemental analysis gave: C(found) 37.5%, H(found) 5.7%, N(found) 3.5%, Na (found) 3.7%. This composition fits to a product with DS = 2.0 in which 50% of CO₂H actually is CO₂Na, which requires C 40.4%, H 5.2.%, N 4.7%, Na 3.8% (for C₂₀H₃₁N₂O₁₇Na, 594.4). Some cross-linked molecules may account for the slightly lower nitrogen-content observed.

The structure of the product with DS = 2 is shown in the Figure below:

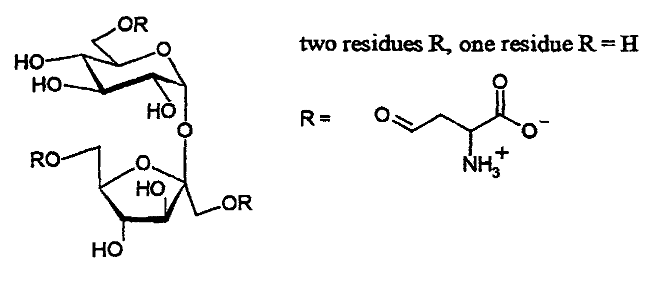

### Example 6:

Sucrose (60.0 g, 0.175 mol) was dissolved in DMSO (150 ml) at 80°C. After cooling to room temperature, sodium acetate (31.6 g, 0.386 mol) was added, followed by maleic anhydride (37.8 g, 0.386 mol) in one portion under stirring. Stirring was continued for 10 min at room temperature and 2 h at 80°C. The product was precipitated with ethyl acetate and pulverised using an Ultra Turrax and then freeze-dried from water. The ¹H NMR spectrum of this product was recorded on a Bruker WT 400 (400 MHz, D₂O). It showed the presence of an isomeric mixture of sucrose maleates: δ = 6.67 and 5.93 ppm (2 multiplets of maleate protons), 5.4 ppm (m, Glc-β-H1). Integration of these signals showed the DS to be ca. 2.0.

### Example 7: (β-Cyclodextrin aspartate)

A) β-Cyclodextrin (Fluka No. 28707, (20.0 g, 0.018 mol) was dissolved in dry DMF (300 ml) at 80°C. After cooling to room temperature, sodium acetate (11.6 g, 0.141 mol, 8.0 equiv.) was added, followed by maleic anhydride (13.8 g, 0.141 mol, 8.0 equiv.). Stirring was continued for and 120 min at 120°C and 2 h at 100°C under nitrogen atmosphere. The next reaction with ammonia could be performed after cooling to 45° in the same flask (B). Alternatively, the intermediate β-CyD maleate could be isolated by stirring in ethyl acetate (2 x 200 ml) and methanol (2 x 100 ml) to yield 30.0 g of the crude product. The ¹H NMR spectrum of this intermediate product was recorded on a Bruker WT 400 (400 MHz, D₂O): δ = 6.5 and 5.7 ppm (2 "d", 2H, maleate), 5.0 ppm (m, 1H, Glc-β-H1), 3.25-4.50 ppm (m, 6 H). Integration of these signals showed the DS to be ca. 0.9-1.0.
B) After cooling to 45°C, the mixture was reacted with an excess of gaseous ammonia (ca. 20.0 g, 1.11 mol, stirring for 2 h). Then the mixture was allowed to stand over night. The viscous honey-like product was pulverised with ethyl acetate and methanol, and dried. The crude yield was 20.6 g. Solubility in water: 1.0 g product gave a clear, viscous solution in 1 ml of water, whereas only up to 18 mg of β-cyclodextrin could be dissolved in 1 ml of water (J.Szejtli, page 357).

The structure of heptakis(6-O-β-aspartoyl)-cyclomaltoheptaose (cyclomaltoheptaose is β-CyD) is shown in the Figure below (DS 1.0):

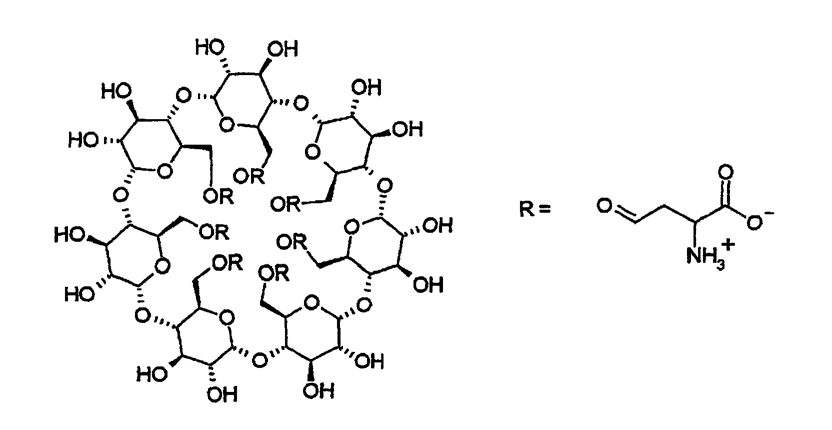

### Example 8:

a) Preparation of a corn seed treatment formulation: 150 g of 90 mesh technical casein (Havero Hoogwegt) was slowly added to 850 ml of water containing 15 ml glycerol (87%, Merck) and 705 g of urea (Merck) while stirring. The pH was adjusted to 9.0 with 25% ammonia solution. Then casein was added very slowly to avoid the formation of lumps (ca. 5-10 g per minute) under stirring and heating at 60°C. During the casein addition, the pH was kept constant at ca. 9.0 with the aqueous ammonia solution. Too much vigorous stirring would cause foaming which would lead to the formation of gas bubbles in the films. On the other hand, too little stirring would cause an inefficient formation of the desired protein dispersion. After complete dispersion of the protein, a 2% milky dispersion of Ca(OH)₂ (1.5 g) was added very slowly under vigorous stirring at 30°C. Then the dispersion was heated to 60°C while stirring. After cooling to ambient temperature, potassium sorbate (2.0 g, Nutrinova GmbH, Frankfurt) was added. The pH value was ca. 8-9. Directly after the preparation of this dispersion, or optionally prior to its application to the seeds, the polyepoxide reagent Kycoat® (Hercules Corp., Siegburg, Germany) was added. The amount of cross-linking reagent was adjusted to 20 wt-% relative to the protein content.
b) Preparation of a seed treatment formulation containing Amisorb®: Amisorb® from Donlar Corporation (Chicago, USA;, a nutrient uptake enhancer) was added by thoroughly mixing the commercially available concentrate (contains ca. 50% of active ingredient) with the dispersion prepared according to a) to obtain the content of 3.8% of Amisorb® by weight.
c) Preparation of a seed treatment formulation containing (l): Compound (I) was prepared according to example 1 and thoroughly mixed with the dispersion prepared according to a) to obtain the content of 4.5% of (I) by weight.
d) Treatment of seeds: An amount of 400 mg (500 µl) of each composition was used to treat each 100 g of corn seeds. In addition, the treatment compositions were mixed with Colanyl Red® (Clariant, Muttenz) to obtain a content of this dye of about 4.5 wt-%. The formulations were applied to the seeds by using the Rotostat M150 machine from J.E. Elsworth Ltd. (Norfolk, UK). Inspection of the treated seeds showed that the coatings were uniformly present all over the seed surface area.
e) Testing of the treated seeds: Plant growth was monitored in 3 groups of planting pots containing 136 plants in each group: Group (1): untreated control; Group (2): treated with formulation (b) containing Amisorb®); Group (3): treated with formulation(c) containing (I).
   Untreated, non-sterilised soil from the farmland located in Frankfurt/Höchst (Germany) was used. All plants received artificial sun light for 8 hours per day and 12.3 ml of water once per day, corresponding to 450 mm/year rainfall, at a temperature range of about 22-27°C. After 14 days the plants were harvested and seedling numbers, length and weight were immediately determined. The results shown in the figure indicate - within reasonable limits of error - that (I) (left bars) provides an about equal benefit (improved seedling vigour parameters) as the bioregulator Amisorb® containing treatment composition.

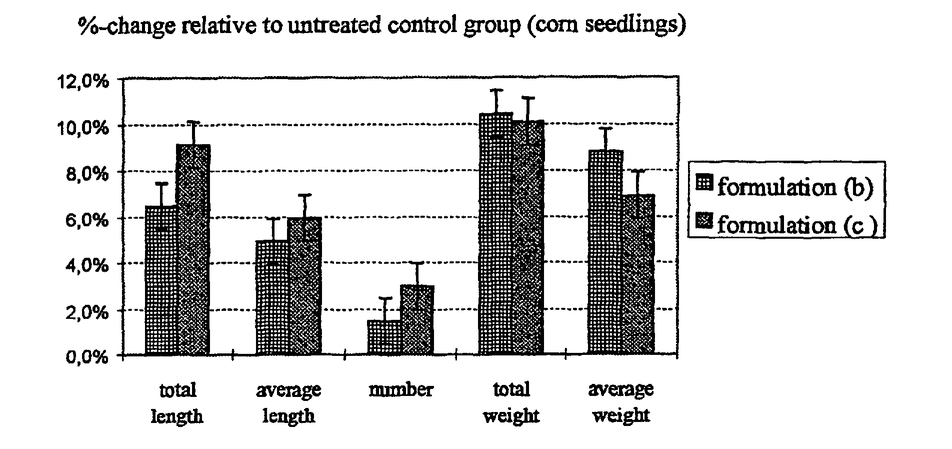

### Example 9:

A wheat seed treatment composition containing (I): A seed treatment formulation was prepared in analogy to example 8 (a). A formulation containing (I) was prepared according to example 8 (c ) by thoroughly mixing with (I) to obtain a content of active ingredient (I) of 5.0% by weight. Wheat seeds (summer wheat, variety *Munk)* were treated with both formulations (see example 1) and plant growth was monitored in 3 groups of planting pots containing 136 plants in each group:

Group (1): untreated control; Group (2): treated with protein formulation only; Group (3): treated with protein formulation containing 5 %-wt of (I). Non-sterilised soil from AgrEvo (Frankfurt, Germany), mixed with 2.4 g of Blaukom fertiliser per kg of soil, was used. All plants received artificial sun light for 8 hours per day and 12.3 ml of water once per day, corresponding to 450 mm/year rainfall, at a temperature range of about 22-27°C. After 14 days the plants were harvested and seedling numbers, length and weight were immediately determined. The results shown in the figure show the improved seedling vigour for the wheat seeds which had been treated with the formulation containing active ingredient (I).

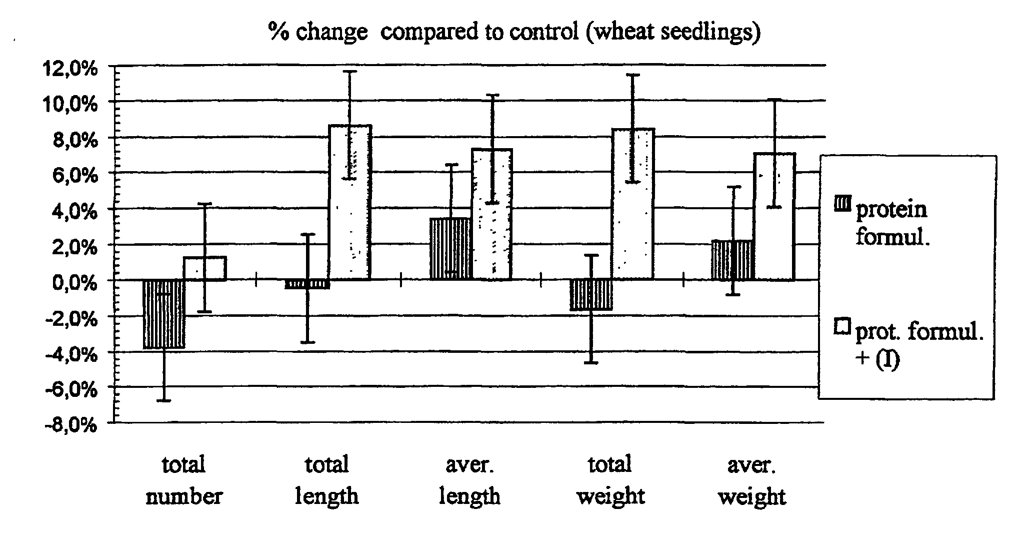

## Claims

1. Polysaccharide aspartate of the general formula (I) 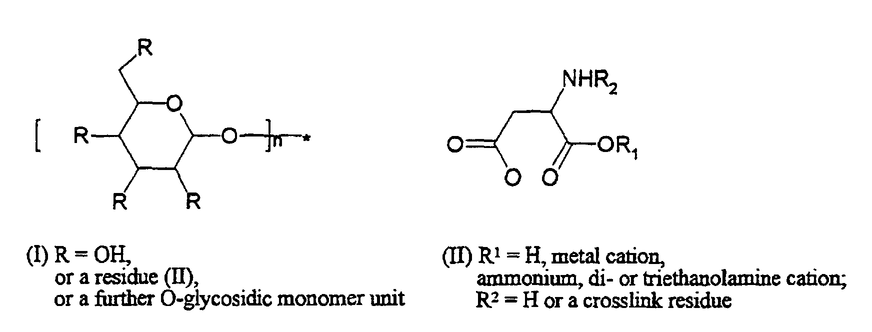 wherein R is hydroxyl, an aspartate residue of formula (II) and/or a glycosidic bond to another monomer sugar unit, and wherein n is greater than one according to the average number of the sugar units given by the parent polysaccharide starting material,
and wherein R¹ is hydrogen, a metal cation, ammonium, di- or triethanolamine cation or a cross-link position to another polysaccharide chain.

2. A polysaccharide aspartate according to claim 1, wherein the degree of substitution (DS) with respect to the ratio of the sugar monomeric units being modified with the residues (II) varies from DS 0.001 to DS 3.0.

3. A polysaccharide aspartate according to any of the preceding claims, wherein the polysaccharide is meant to include the naturally occurring products made up from two to 100 000 of monosaccharide units per molecule, preferably a starch, maltose, glycogen, dextrin, amylose, amylopectin, dextrin, cyclodextrin, sucrose, lactose, dextran, xanthan, cellulose or chitin, most preferably a starch, dextrin, cyclodextrin, cellulose or chitin.

4. A method to prepare polysaccharide aspartates of formula (I), 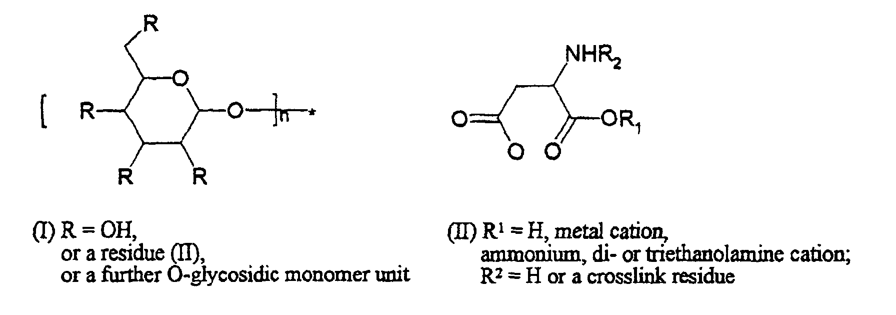 wherein R is hydroxyl, an aspartate residue of formula (II) and/or a glycosidic bond to another monomer sugar unit, and wherein n is greater than one according to the average number of the sugar units given by the parent polysaccharide starting material, and wherein R¹ is hydrogen, a metal cation, ammonium, di- or triethanolamine cation or a cross-link position to another polysaccharide chain; by first reacting a polysaccharide with maleic anhydride and secondly further reacting the intermediate formed with 1-5 equivalents of ammonia (calculated relative to the maleate units), wherein the reaction is carried out either in an organic solvent or solvent free and the temperature of the reaction mixture is in the range of 15-65°C.

5. A method according to claim 4, wherein the reaction with ammonia is carried out in the same solvent being used to prepare the intermediate polysaccharide maleate.

6. A method according to any of the preceding claims 4 or 5, wherein the reaction is carried out at room temperature and the reaction time lies in the range of 1-48 hours.

7. A method according to any of the preceding claims 4-6, wherein the degree of substitution (DS) with respect to the ratio of the sugar monomeric units being modified with the residues (II) varies from DS 0.001 to DS 3.0.

8. A method according to any of the preceding claims 4-7, wherein the first reaction between the maleic anhydride and the polysaccharide is carried out in a non-aqueous solvent and an equimolar amount (relative to maleic anhydride) of a basic metal salt MX (M= alkaline metal, X= anion) is added to the reaction mixture.

9. The use of the polysaccharide aspartate of formula (I) according to claim 1, as a chromatography support, an ion exchanger material, a metal ion absorbent material or as a drug delivery device or in seed treatment or soil treatment compositions.

10. A soil treatment composition containing 0.001-5 wt.-% of compound (I).

11. A seed treatment composition containing 0.01-35 wt.-% of compound (l).

## Patentansprüche

1. Polysaccharidaspartate der allgemeinen Formel (I) 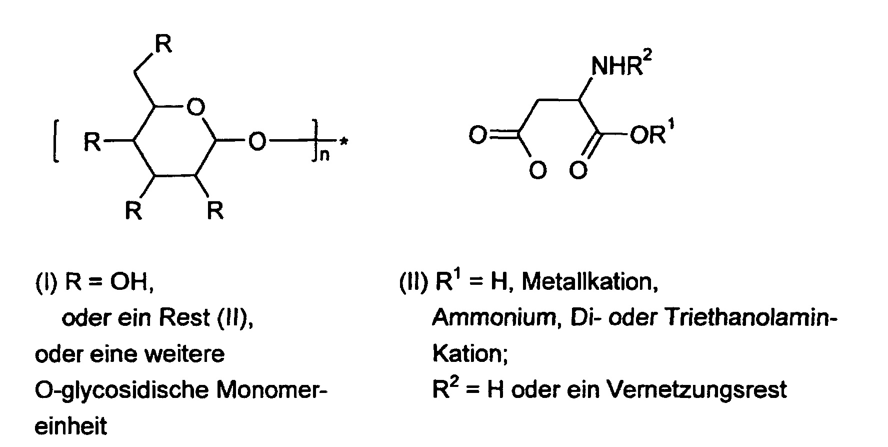 wobei R Hydroxyl, ein Aspartatrest der Formel (II) und/oder eine glycosidische Bindung zu einer anderen Zuckermonomereinheit ist, und wobei n größer als 1 ist und der durch das Polysaccharid-Ausgangsmaterial gegebenen durchschnittlichen Anzahl von Zuckereinheiten entspricht,
und wobei R¹ ein Wasserstoffatom, ein Metallkation, Ammonium, ein Di- oder Triethanolamin-Kation oder eine Vemetzungsstelle zu einer anderen Polysaccharidkette ist.

2. Polysaccharid-Aspartat nach Anspruch 1, wobei der Substitutionsgrad (DS) in Bezug auf das Verhältnis der Zuckermonomereinheiten, die mit den Resten (II) modifiziert sind, von DS 0,001 bis DS 3,0 variiert.

3. Polysaccharidaspartat nach einem der vorhergehenden Ansprüche, wobei das Polysaccharid zu verstehen ist als die natürlich vorkommenden, aus von zwei bis 100 000 Monosaccharideinheiten pro Molekül bestehenden Produkte einschließend, vorzugsweise eine Stärke, Maltose, Glycogen, Dextrin, **Amylose, Amylopektin, Cyclodextrin, Sucrose, Lactose, Dextran, Xanthan,** Cellulose oder Chitin, am bevorzugtesten eine Stärke, Dextrin, Cyclodextrin, Cellulose oder Chitin.

4. Verfahren zur Herstellung von Polysaccharidaspartaten der Formel (l), 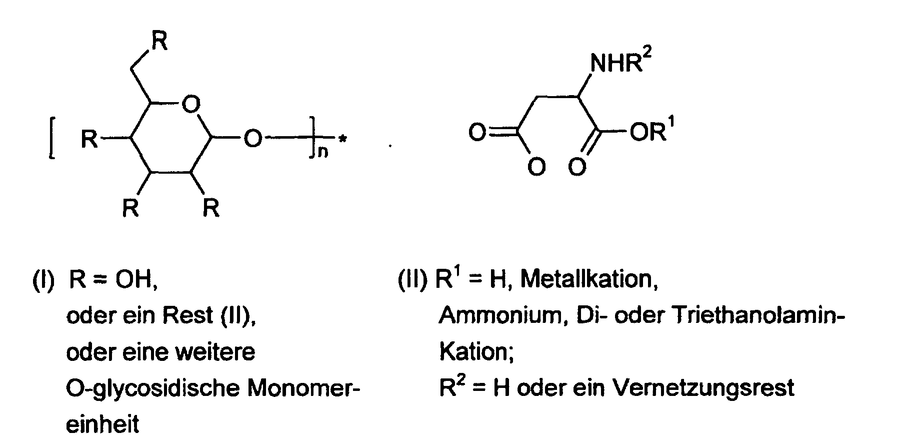 wobei R Hydroxyl, ein Aspartatrest der Formel (ll) und/oder eine glycosidische Bindung zu einer anderen Zuckermonomereinheit ist, und wobei n größer als 1 ist und der durch das Stammpolysaccharid-Ausgangsmaterial gegebenen durchschnittlichen Anzahl von Zuckereinheiten entspricht, und wobei R¹ ein Wasserstoffatom, ein Metallkation, Ammonium, ein Di- oder Triethanolamin-Kation oder eine Vernetzungsstelle zu einer anderen Polysaccharidkette ist; indem zuerst ein Polysaccharid mit Maleinsäureanhydrid umgesetzt wird, und als zweites das gebildete Zwischenprodukt weiter mit 1-5 Äquivalenten Ammoniak umgesetzt wird (berechnet mit Bezug auf die Maleateinheiten), wobei die Reaktion entweder in einem organischen Lösemittel oder lösemittelfrei ausgeführt wird, und die Temperatur der Reaktionsmischung in dem Bereich von 15-65°C ist.

5. Verfahren nach Anspruch 4, wobei die Reaktion mit Ammoniak in dem selben Lösemittel ausgeführt wird, das verwendet wird, um das Polysaccharidmaleat-Zwischenprodukt herzustellen.

6. Verfahren nach einem der vorhergehenden Ansprüche 4 oder 5, wobei die Reaktion bei Raumtemperatur durchgeführt wird, und die Reaktionszeit in dem Bereich von 1-48 Stunden liegt.

7. Verfahren nach einem der vorhergehenden Ansprüche 4 bis 6, wobei der Substitutionsgrad (DS) in Bezug auf das Verhältnis der Zuckermonomereinheiten, die mit den Resten (ll) modifiziert sind, von DS 0,001 bis DS 3,0 variiert.

8. Verfahren nach einem der vorhergehenden Ansprüche 4 bis 7, wobei die erste Reaktion zwischen dem Maleinsäureanhydrid und dem Polysaccharid in einem nichtwässrigen Lösemittel durchgeführt wird und eine äquimolare Menge (bezüglich des Maleinsäureanhydrids) eines basischen Metallsalzes MX (M = Alkalimetall, X = Anion) der Reaktionsmischung zugesetzt wird.

9. Verwendung des Polysaccharidaspartates der Formel (1) nach Anspruch 1 als Chromatographieträger, lonenaustauschermaterial, Adsorptionsmaterial für Metallionen oder als ein Medikamentfreisetzungsmittel, oder in Zusammensetzungen zur Saatgutbehandlung oder zur Bodenbehandlung.

10. Zusammensetzung zur Bodenbehandlung, enthaltend 0,001-5 Gew.-% der Verbindung (l).

11. Zusammensetzung zur Saatgutbehandlung, enthaltend 0,01-35 Gew.-% der Verbindung (l).

## Revendications

1. Aspartate de polysaccharide répondant à la formule générale (I) 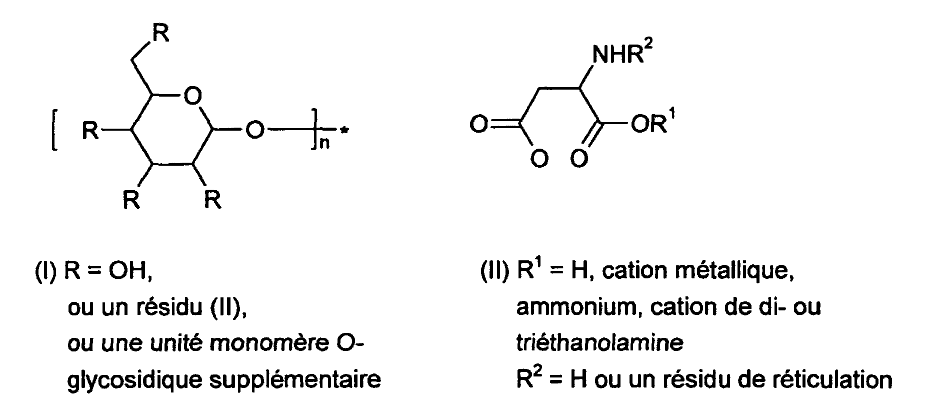 dans laquelle R représente un groupe hydroxyle, un résidu aspartate répondant à la formule (ll) et/ou une liaison glycosidique avec une autre unité saccharique monomère, et dans laquelle n est plus grand que 1 selon le nombre moyen d'unités sacchariques donné du précurseur du polysaccharide parent,
et dans laquelle R¹ représente un atome d'hydrogène, un cation métallique, un groupe ammonium, un cation di- ou triéthanolamine ou une position de réticulation avec une autre chaîne polysaccharidique.

2. Aspartate de polysaccharide selon la revendication 1, dans lequel le degré de substitution (DS), eu égard au rapport des unités monomériques sacchariques modifiées avec les résidus (ll), varie de 0,001 DS à 3,0 DS.

3. Aspartate de polysaccharide selon l'une quelconque des revendications précédentes, dans lequel le polysaccharide est prévu pour inclure les produits existants naturellement composés de deux à 100 000 unités de monosaccharides par molécule, de préférence un amidon, un maltose, un glycogène, une dextrine, un amylose, une amylopectine, une dextrine, une cyclodextrine, un saccharose, un lactose, une dextrane, un xanthane, une cellulose ou une chitine, de manière préférée entre toutes, un amidon, une dextrine, une cyclodextrine, une cellulose ou une chitine.

4. Procédé pour préparer des aspartates polysaccharidiques répondant à la formule (I), 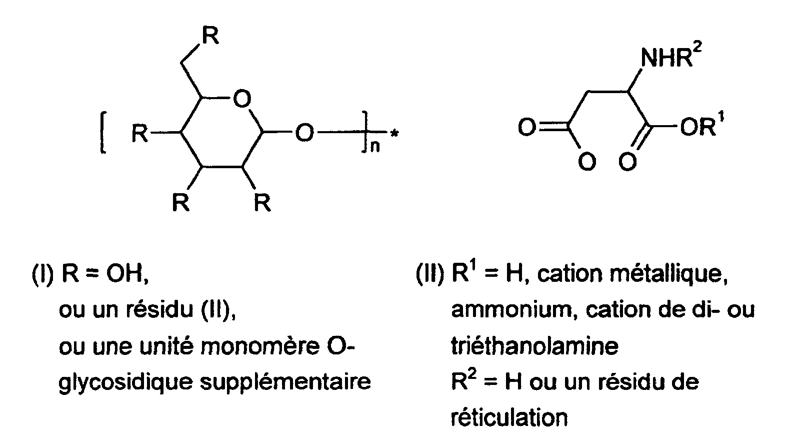 dans laquelle R représente un groupe hydroxyle, un résidu aspartate répondant à la formule (ll) et/ou une liaison glycosidique avec une autre unité saccharique monomère, et dans laquelle n est plus grand que 1 selon le nombre moyen d'unités sacchariques donné du précurseur du polysaccharide parent, et dans laquelle R¹ représente un atome d'hydrogène, un cation métallique, un groupe ammonium, un cation di- ou triéthanolamine ou une position de réticulation avec une autre chaîne polysaccharidique; en faisant réagir, dans un premier temps, un polysaccharide avec de l'anhydride maléique et, dans un deuxième temps, en faisant ultérieurement réagir l'intermédiaire formé avec 1-5 équivalents d'ammoniac (calculés par rapport aux unités de maléate), dans laquelle la réaction est réalisée soit dans un solvant organique soit sans solvant et la température du mélange réactionnel est dans la plage de 15-65°C.

5. Procédé selon la revendication 4, dans lequel la réaction avec l'ammoniac est réalisée dans le même solvant que celui utilisé pour préparer le maléate polysaccharidique intermédiaire.

6. Procédé selon l'une quelconque des revendications précédentes 4 ou 5, dans lequel la réaction est réalisée à température ambiante et le temps de réaction demeure dans la plage de 1-48 heures.

7. Procédé selon l'une quelconque des revendications précédentes 4 à 6, dans lequel le degré de substitution (DS), eu égard au rapport des unités monomériques sacchariques modifiées avec les résidus (ll), varie de 0,001 DS à 3,0 DS.

8. Procédé selon l'une quelconque des revendications précédentes 4 à 7, dans lequel la première réaction entre l'anhydride maléique et le polysaccharide est réalisée dans un solvant non aqueux, et une quantité équimolaire (par rapport à l'anhydride maléique) d'un sel métallique basique MX (M = métal alcalin, X = anion) est ajoutée au mélange réactionnel.

9. Utilisation de l'aspartate de polysaccharide répondant à la formule (I) selon la revendication 1, en tant que support de chromatographie, matière échangeuse d'ions, matière absorbant les ions métalliques ou en tant que dispositif de relargage de médicaments ou dans des compositions de traitement des graines ou de traitement des sols.

10. Composition de traitement des sols contenant 0,001-5 % en poids du composé (I).

11. Composition de traitement des graines contenant 0,01-35 % en poids du composé (1).
